# EUROPEAN PATENT APPLICATION

(11) **EP 4 454 538 A1**
(43) Date of publication of application: **30.10.2024**
(21) Application number: 24171256.1
(22) Date of filing: 19.04.2024
(51) Int. Cl.: A61B 1/00, A61B 1/12, A61B 1/313, A61B 17/34, A61B 1/015, A61B 90/70

(54) **ACCESS ASSEMBLYS FOR SURGICAL INSTRUMENT**

(30) Priority: 26.04.2023 CN 202310494463
(71) Applicant: NINGBO HITCM MEDICAL DEVICES CO., LTD., Ningbo, Zhejiang 315000 (CN)
(72) Inventor: DING, Weijiang, Ningbo, Zhejiang, 315000 (CN); YUAN, Peng, Ningbo, Zhejiang, 315000 (CN); CAI, Zhefeng, Ningbo, Zhejiang, 315000 (CN)
(74) Representative: Viering, Jentschura & Partner mbB Patent- und Rechtsanwälte

(57) **Abstract**

The present disclosure relates to an access assembly for a surgical instrument, including: a tube member, a proximal sealing device connecting the surgical instrument to a proximal end of the tube member in a sealed manner, a distal end member connected with the tube member, a fluid supply passage for providing the fluid to the access assembly and a fluid discharge passage configured for the access assembly to discharge the fluid. The distal end member includes: a tubular housing connected with a distal end of the tube member; and a plurality of sealing valves arranged at a distal end of the tubular housing and configured to be openable and closable to open and seal a distal end of the access assembly. At least one of the plurality of sealing valves includes a nozzle for ejecting a fluid, a fluid inlet for receiving the fluid, and a distal end member fluid channel for fluidly communicating the fluid inlet and the nozzle; the nozzle is arranged to eject the fluid in a proximal direction of the access assembly, the ejection direction of the nozzle forms an angle α with respect to a longitudinal axis of the distal end member; the fluid discharge passage includes an outlet and an inlet, the inlet is arranged at the proximal end of the tube member.

## Description

### TECHNICAL FIELD

The present disclosure relates to an access assembly, in particular to an access assembly for a surgical instrument.

### BACKGROUND

During the surgical procedure, a surgeon usually needs to diagnose and/or treat relevant organs in a patient's body by means of surgical instruments (such as forceps, graspers, cutters, applicators, etc.), which must be introduced into the patient's body by means of a cannula. However, operatively executive parts of the surgical instruments introduced into the patient's body are easily adhered with condensates, tissues, bloods and other body fluids in the body cavity during the diagnosis and/or treatment, which adversely affects or impairs the effectiveness of diagnosis and/or treatment. Therefore, it is very important to keep the relevant surgical instruments, especially the lens of the endoscope, clean during the surgery. Conventionally, the surgeon (e.g., an endoscopist) withdraws the endoscope from an incision on the patient's body via an access assembly, cleans the lens with pre-prepared physiological saline at a body temperature, then wipes the endoscope with a disinfectant such as iodophor and reinserts the endoscope into the incision on the patient's body via the access assembly. During the surgical procedure, the time required to clean the lens may increase the total time of the surgery and the amount of time the patient needs to remain anesthetized; furthermore, since the endoscope is repeatedly withdrawn from and inserted into the incision on the patient's body, this may result in increased risk of infection and also prolong the recovery time. Therefore, there is a need in the art for an improved access assembly that can easily and quickly clean the lens of an endoscope during a surgery without introducing additional irrigation liquid or dirt into the body cavity of a patient.

The prior art discloses an access assembly for an endoscope, including a tube member for receiving the endoscope and a distal end member connected with a distal end of the tube member. The distal end member includes a plurality of sealing valves, and at least one of the plurality of sealing valves includes a fluid inlet, a nozzle, and a distal end member fluid channel for fluidly communicating the fluid inlet with the nozzle. The nozzle is close to a central axis of the distal end member and is arranged in parallel with the central axis of the distal end member, and the jet flows are vertically ejected onto the lens of the endoscope. Although the access assembly can clean the lens of the endoscope to a certain extent, since the nozzle is close to the central axis of the distal end member, a longer fluid channel exists between the nozzle and the fluid inlet, and the longer fluid channel, in turn, will lead to a longer mold insert for producing the distal end member correspondingly; as a result, the mold is more easily deformed when injection-molding the distal end member, which ultimately shortens the service life of the mold and reduces the production yield of the distal end member of the access assembly. In addition, since the nozzle is close to the central axis of the distal end member, there is a certain probability that the nozzle still ejects water when the water on the lens is scraped by a scraping device. Therefore, the water on the lens cannot be completely removed.

### SUMMARY

The technical problem to be solved by the present disclosure is to provide an access assembly, which can achieve an improved cleaning effect, especially an effect of cleaning a distal end part of a surgical instrument at any angle in a cavity of the access assembly by setting a position of the nozzle.

An access assembly for a surgical instrument, including: a tube member extending along a longitudinal axis of the access assembly and configured to receive the surgical instrument; a proximal sealing device connecting the surgical instrument to a proximal end of the tube member in a sealed manner; and a distal end member connected with the tube member. The distal end member includes: a tubular housing connected with a distal end of the tube member; and a plurality of sealing valves arranged at a distal end of the tubular housing. The plurality of sealing valves are structured to be openable and closable to open and seal a distal end of the access assembly; at least one of the plurality of sealing valves includes a nozzle for ejecting a fluid, a fluid inlet for receiving the fluid, and a distal end member fluid channel for fluidly communicating the fluid inlet and the nozzle. The nozzle is configured to eject the fluid towards a proximal direction of the access assembly directionally, and an ejection direction of the nozzle has an angle a with respect to a longitudinal axis of the distal end member. The access assembly further includes a fluid supply passage for providing the fluid to the access assembly; and a fluid discharge passage for the access assembly to discharge the fluid. The fluid discharge passage includes an outlet and an inlet, and the inlet of the fluid discharge passage is arranged at the proximal end of the tube member. A value of the angle α is less than or equal to 60 degrees, preferably, the value of angle α is less than or equal to 15 degrees.

This technical problem is solved by an access assembly for a surgical instrument according to the present disclosure, in which the nozzle is oriented at an angle α relative to the longitudinal axis of the distal end member, and when the value of the angle α is equal to 15 degrees, the nozzle is directed to the inlet of the fluid discharge passage of the access assembly. The value of the angle α is preferably less than or equal to 15 degrees, which is particularly beneficial to performing cleaning at any position and any angle in the cavity. Through the design of the nozzle according to the present disclosure, the jet flow ejected from the nozzle can effectively cover the entire effective cleaning space of the access assembly, especially the entire tube member section from the inlet of the fluid discharge passage to the distal end member, so as to realize the cleaning at any position and any angle in the cavity of the tube member section. In a case that the value of the orientated angle α of the nozzle is greater than 15 degrees, the jet flow can only cover part of the effective cleaning space of the access assembly, especially part of the tube member section from the inlet of the fluid discharge passage to the distal end member. In view of this, when the surgical operation is changed from the working state to the cleaning state, the distal end part of the surgical instrument, especially the lens of the laparoscope, must be placed in a specific position to be cleaned, which is obviously not beneficial to simplifying the cleaning operation of the access assembly. In a case that the value of angle α is greater than 60 degrees, the fluid ejected from the nozzle 16 cannot be ejected onto the lens of the endoscope if the lens is facing away from the nozzle 16.

According to a developed solution of the present disclosure, the sealing valveis provided with a scraping device for scraping a distal end part of the surgical instrument. The nozzle is located away from the scraping device and is arranged on at least one of the plurality of sealing valves; and the scraping device is at least partially arranged at a central axis of the distal end member. If the nozzle is arranged close to the longitudinal axis of the distal end member, especially as in the prior art, it is easy to leave water on the distal end part of the surgical instrument when the surgical instrument having been introduced is cleaned by the access assembly. As comparison, if the nozzle is arranged on one of the sealing valves and located away from the longitudinal axis of the distal end member, it is not easy to leave water on the distal end part of the surgical instrument, especially on the lens of an endoscope, after the completion of the cleaning of the surgical instrument. Therefore, the nozzle arranged according to the present disclosure can obtain better cleaning effect. At the same time, due to the arrangement of the nozzle on one of the sealing valves and away from the longitudinal axis of the distal end member according to the present disclosure, the fluid passage of the distal end member according to the present disclosure is also significantly shorter, which in turn shortens the mold insert required for producing the distal end member. The shortened mold insert makes the mold used for producing the distal end member not easy to be deformed, which significantly improves the production yield of the distal end member of the access assembly and hence the production yield of the access assembly.

According to a further developed solution of the present disclosure, a radial distance between a center point of the nozzle and a central axis of the distal end member is greater than or equal to one quarter of an outer diameter of the tubular housing, which is particularly beneficial to obtaining a good cleaning effect and maintaining a high production yield.

According to a further developed solution of the present disclosure, a distance between a proximal edge of the nozzle and a base of the distal end member is greater than a distance between a proximal edge of the scraping device and the base of the distal end member. During a process that the lens of the endoscope is retracted from the distal end member proximally, the sealing valve is at the beginning of an open state. At this time, due to the action of a negative pressure, gas in the abdominal cavity will flow into the tube member of the access assembly through a gap, and the water on the distal end part of the surgical instrument will be taken away with the airflow. If there is a lot of water on the distal end part of the surgical instrument, the suction effect of the negative pressure will not be enough to remove all the water, resulting in water droplets remaining on the lens. Through this solution, the nozzle can be blocked before the surgical instrument is in contact with the scraping device, especially the scraper, during the surgical instrument moving distally, thus reducing the amount of water ejected onto the distal end part of the surgical instrument, especially onto the lens of the endoscope. As a result, less water needs to be taken away by the suction effect of the negative pressure, and water droplets are not easy to remain on the distal end part of the surgical instrument, thereby improving the effect of removing the water droplets.

In a still further developed solution, a distance B between an inner edge of the nozzle away from the central axis of the distal end member and an outer wall of the tubular housing is greater than an inner diameter A of the nozzle. Through this technical solution, the wall-adhering phenomenon can be well avoided. The wall-adhering phenomenon refers to that the jet ejected from the nozzle is not in an ejected state but flows along the inner wall of the tube member of the access assembly. The occurrence of this phenomenon will result in that the jet flow cannot be effectively ejected onto the distal end part of the surgical instrument, thus adversely affecting the cleaning effect.

In a further developed solution of the present disclosure, it is advantageously provided that the scraping device protrudes proximally so that it can be in contact with the distal end part of the surgical instrument, especially under a negative pressure, when the surgical instrument passes through the sealing valve.

In a further improved solution of the present disclosure, it is advantageously provided that the plurality of sealing valves are arranged along a circumferential direction of the tubular housing, and each of the plurality of sealing valves includes two radial edges and one circumferential edge, and the scraping device protrudes proximally. The extent of protruding depends on an angle β formed between the radial edge of the sealing valve and the longitudinal axis of the distal end member, which angle β is suitable for the negative pressure provided by means of the access assembly.

According to a developed design of the present disclosure, it's provided that each of the plurality of sealing valves includes a ridge extending radially along the tubular housing and surfaces on both radial sides of the ridge; the nozzle is arranged on one of a plurality of the ridges; and the distal end member fluid channel extends in a plane bounded by the longitudinal axis of the distal end member and the ridge. If the nozzle is arranged at other positions of the sealing valve, the closing actions of the sealing valves will be inconsistent when they close, that is, the sealing valve provided with the nozzle will close later in time sequence, thus forming a short-time gap caused by the difference in closing time. Through this developed solution, the occurrence of such gap can be basically eliminated, so that more reliable sealing can be realized when cleaning the surgical instrument.

According to the present disclosure, it is particularly advantageous that the scraping device includes at least one boss which protrudes proximally from the sealing valve and is arranged on the sealing valve along at least one radial edge of the sealing valve; and the scraping device further includes at least one scraper, which protrudes proximally from a part of the boss close to the longitudinal axis of the distal end member.

According to a further developed solution of the present disclosure, the distal end member further has at least one elastic piece protruding proximally from the tubular housing. The elastic piece is structured to form a fluid channel together with the tube member, and has a first state and a second state. The elastic piece is bent radially outwards relative to the longitudinal axis of the distal end member in the first state, and the elastic piece is bent radially inwards relative to the longitudinal axis of the distal end member in the second state. Due to the different states of the elastic piece, a cross-sectional area of the fluid channel is varied. When the elastic piece is in the first state, the cross-sectional area of the fluid channel becomes larger, which makes the fluid resistance become smaller and the flow rate become faster under the same negative pressure; correspondingly, the impulse force becomes greater, so that the cleaning effect of the access assembly according to the present disclosure is better.

According to a further developed solution of the present disclosure, the distal end member is connected with at least one elastic piece, and the elastic piece is structured to form at least part of the fluid supply passage, together with a part of the tube member; and a distal end of the part of the fluid supply passage is communicated with the distal end member fluid channel.

If the above-mentioned surgical instrument is an endoscope, the distal end part of the surgical instrument is a lens of the endoscope.

### BRIEF DESCRIPTION OF DRAWINGS

The above-mentioned characteristics, features and advantages of the present disclosure and the way to realize them can be more clearly understood by combining with the following description of non-limiting embodiments set forth in more detail with reference to the accompanying drawings. As shown in the accompanying drawings:
Fig. 1 is a lateral cross-sectional view of an access assembly according to the present disclosure;
Fig. 2 is a lateral top perspective view of a distal end member of an access assembly according to the present disclosure;
Fig. 3 is a cross-sectional view of a distal end member taken along line A-A in Fig. 2 according to an embodiment of the present disclosure;
Fig. 4 is a detailed view of a distal end member represented by a dotted circle in Fig. 3;
Fig. 5 is a top view of a distal end member according to the present disclosure;
Fig. 6A is a cross-sectional view of a distal end member taken along line B-B in Fig. 2 according to an embodiment of the present disclosure;
Fig. 6B is a cross-sectional view of another embodiment of a distal end member according to Fig. 6A;
Fig. 7A is a top view of an elastic piece of an access assembly according to the present disclosure in a first state;
Fig. 7B is a top view of an elastic piece of an access assembly according to the present disclosure in a second state;
Fig. 8A is a lateral cross-sectional view of an access assembly in a cleaning state; and
Fig. 8B is a lateral cross-sectional view of an access assembly in a cleaning state.

### DETAILED DESCRIPTION

The following embodiments are preferred implementations of the present disclosure. In these embodiments, the individual components of the described embodiments form individual features of the present disclosure which are considered separately and independently from each other. These individual components also expand the present disclosure independently from each other, and hence can also form a part of the present disclosure separately, or in other forms different from the illustrated combination(s). In addition, the described implementations can also be supplemented by other features of the present disclosure already described.

Before specifically explaining the various aspects of the access assembly and various features thereof, it should be noted that the various aspects disclosed herein are not limited in their application or usage to the details of construction and arrangement of the components and parts illustrated in the drawings and description. Rather, the disclosed device may be located or combined into other devices, variations and modifications thereof, and may be practiced or performed in various ways. Accordingly, aspects of features of the distal end member and the access assembly disclosed herein are illustrative in nature and are not meant to limit their scope or application. Furthermore, unless otherwise specified, the terms and expressions used herein have been chosen for the convenience of the reader to understand various aspects of the characteristics of the distal end member and the access assembly and are not intended to limit the scope thereof. Furthermore, it should be understood that any one or more components of the distal end member and the access assembly, expressions thereof, and/or examples thereof may be combined with any one or more other components, expressions thereof, and/or examples thereof without limitative.

A distal end member of an access assembly for a surgical instrument and the access assembly are described in details with reference to the accompanying drawings, in which the same reference numerals refer to the same or corresponding elements in each of the above views. As used herein, the technical term "surgical instrument" refers to any surgical instrument that can be introduced into a subject's body through an access assembly such as a puncture device, and specifically refers to an endoscope in the non-limiting embodiments of the present disclosure; the terms "distal" and "distal side" refer to a direction (for example, the downward direction as illustrated in the drawings) in which the surgical instrument, a portion or a component of the access assembly are farther away from an operator (for example, a surgeon); further, the term "distal direction" refers to a direction along which the surgical instrument, especially the endoscope, is inserted, and the term "distal end" refers to an end away from the surgical instrument, especially the endoscope, relative to the operator (e.g., the surgeon); on the contrary, the terms "proximal" and "proximal side" refer to a direction (for example, the upward direction as illustrated in the drawings) in which the endoscope, a portion or a component of the access assembly are closer to the operator, further, the term "proximal direction" refers to a direction along which the surgical instrument, especially the endoscope, is withdrawn, and the term "proximal end" refers to an end closer to the surgical instrument, especially the endoscope, relative to the operator (e.g., the surgeon). Additionally, in general conception, the term "endoscope" can be used interchangeably with any other equipment such as laparoscope, arthroscope, gastrointestinal endoscope, laryngo-bronchial endoscope, etc. for observing the body cavity of a patient through an incision or a cannula with small diameter. As used herein, the term "about" means that the value is approximate and that small changes will not significantly affect the practice of the disclosed aspects of the present disclosure. Where a numerical limitation is used, "about" means that the value may vary ±10% and still be within the scope of the disclosure, unless the context indicates otherwise.

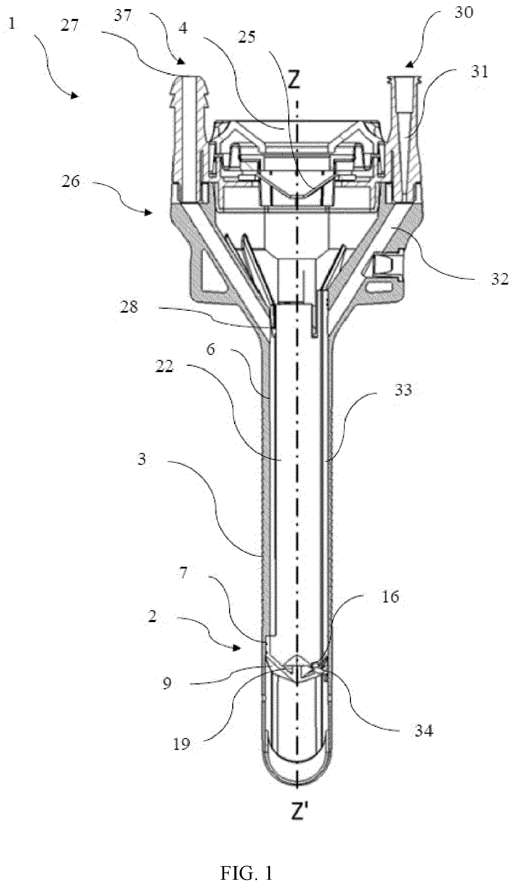

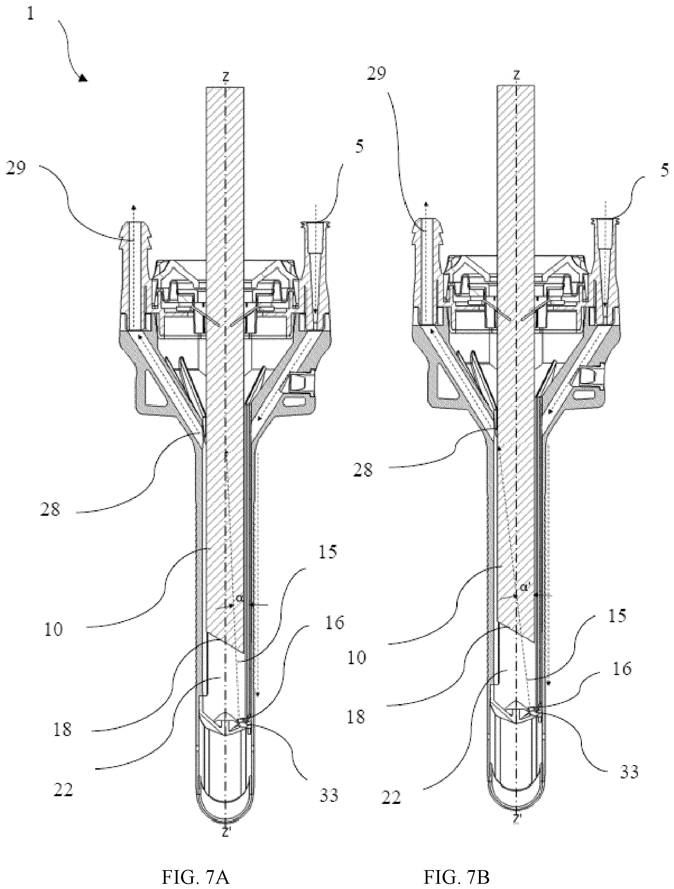

As shown in Fig. 1, the access assembly 1 includes a tube member 3, a proximal sealing device 4 for sealing the access assembly 1 with a surgical instrument at a proximal end, a distal end member 2 for sealing the access assembly 1 at a distal end, and a fluid supply passage 30 for providing a fluid to the access assembly 1. In this embodiment, the tube member 3 is designed as a cannula of a puncture device, and the proximal sealing device 4 is arranged at a proximal end of the tube member 3. When the surgical instrument is inserted from a proximal end of the access assembly 1 and pushed to a distal end of the access assembly 1, the proximal sealing device 4 seals the access assembly 1 with the surgical instrument. The distal end member 2 is designed as a W-shaped sealing member. The distal end member 2 is installed at a distal end position of an inner wall 6 of the tube member 3, and can be configured such that when (as shown in Figs. 7A-7B) the surgical instrument 10, especially the endoscope, passes through the tube member 3, the distal end member 2 abuts against a side wall of the surgical instrument 10, partially opens and forms a seal with this side wall so as to keep the body cavity of the patient isolated from a cavity 22 of the access assembly 1 during the operation of the surgical instrument 10, especially for observing a surgical field.

The tube member 3 is structured to extend along a central axis Z-Z' of the access assembly 1 and configured to receive the surgical instrument 10. The tube member 3 includes a substantially conical-shaped conical portion at its proximal side for gripping and engaging with a proximal sealer 25, and a substantially cylindrical-shaped cylindrical portion at its distal side.

In this embodiment, the distal end member 2 includes a tubular housing 7 and a plurality of sealing valves 9; the tubular housing 7 is structured to be connected with the distal end of the access assembly 1 at a proximal end of the tubular housing 7. An inner diameter of the inner wall 6 of the cylindrical portion of the tube member 3 corresponds to (e.g., substantially equal to or slightly smaller than) an outer diameter of an outer wall 8 of the tubular housing 7 of the distal end member 2, so that a good sealing effect is achieved between the distal end member 2 and the tube member 3.

In this embodiment, four sealing valves 9 of the distal end member 2 are arranged at a distal end of the tubular housing 7, and are structured to be openable and closable so as to open and in particular to unidirectionally seal the distal end of the access assembly 1. At least one of the four sealing valves 9 includes a nozzle 16 for ejecting fluid 15 towards a proximal direction of the access assembly, a fluid inlet for receiving the fluid, and a distal end member fluid channel 34 for fluidly communicating the fluid inlet and the nozzle 16. A scraping device 19 for scraping the distal end part 18 of the surgical instrument 10 is arranged on the four sealing valves 9. Among them, the distal end part 18 is preferably a lens of the surgical instrument.

Under the condition that the plurality of sealing valves are closed and/or under the condition that the sealing valves are opened and sealed with an outer wall of the surgical instrument, the plurality of sealing valves only unidirectionally prevent the fluid from passing therethrough. The distal end member 2 may allow fluid (e.g., CO₂ for artificial pneumoperitoneum) to pass from the outside (for example, a patient's body cavity) of the access assembly 1 so as to enter the cavity 22 of the access assembly 1 (as shown in Figs. 7A-7B), but does not allow fluid to leave the cavity 22 of the access assembly 1 to the outside of the access assembly 1, for example, the patient's body cavity, under a pressure difference in a direction from the outside of the access assembly 1 pointing to the cavity 22 of the access assembly 1.

The proximal sealing device 4 includes a proximal sealer 25, so as to form an additional seal with an outer wall of the surgical instrument 10. For this purpose, the proximal sealer 25 abuts against the outer wall of the surgical instrument 10 when the surgical instrument 10 passes through the proximal sealing device 4.

The inner wall 6 of the tube member 3, a distal surface of the proximal sealer 25 and a proximal surface of the distal end member 2 collectively define the cavity 22 of the access assembly 1, so as to form an interior space which is generally isolated from the external air at the proximal side of the access assembly 1 and the body cavity of the patient at the distal side of the access assembly 1. Furthermore, the pressure of the patient's body cavity can be made higher than that of the cavity 22 of the access assembly 1 by means of artificial pneumoperitoneum, so as to prevent substances within the cavity 22 of the access assembly 1 from entering the patient's body cavity through the distal end member 2.

The access assembly 1 includes a fluid discharge passage 37. The access assembly 1 has a vacuum suction interface 27 (also referred to as an outlet 27 of the fluid discharge passage 37) for connecting to a vacuum suction source (such as a vacuum pump, a vacuum source used as a hospital infrastructure, etc.), a vacuum suction port 28 (also referred to as an inlet 28 of the fluid discharge passage 37), and a vacuum channel (also referred to as the fluid discharge passage 37) that fluidly communicates the vacuum suction interface 27 with the vacuum suction port 28. The vacuum suction port 28 is located at a proximal end of the cavity 22 of the access assembly 1 to suck out the cleaning fluid and dirt from the cavity 22. The cleaning fluid and dirt within the cavity 22 of the access assembly 1 leave the access assembly 1 through the vacuum suction port 28 and the vacuum suction interface 27 along the vacuum channel, and the direction and path thereof are shown by the dashed arrow 29 in Fig. 7A or 7B. Although the vacuum suction port 28 is located at a relatively proximal position in the tube member 3 in this embodiment, its position is not limited to this, that is, it can be arranged at any suitable position within the tube member 3. It should be understood that during a surgery performed by using the access assembly 1 and the surgical instrument 10, depending on different working angles required by the surgical instrument 10 (for example, an upright orientation, an inverted orientation, a horizontal orientation, an obliquely downward orientation, an obliquely upward orientation, etc.), the access assembly 1 is also arranged at an angle as required correspondingly. Accordingly, the cleaning fluid and the dirt may be accumulated at a proximal or distal side within the cavity 22 of the access assembly due to gravity. In this case, the vacuum suction port may be correspondingly arranged at a proximal or distal side within the tube member 3 to facilitate the effect of sucking fluid and dirt out of the cavity 22.

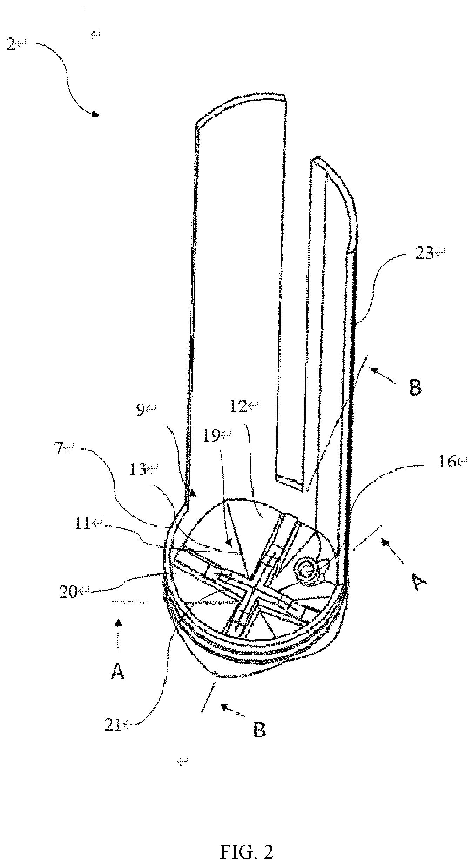

The access assembly 1 includes a fluid supply passage 30, a proximal end of the fluid supply passage 30 is provided with a fluid inlet 5 for receiving a fluid, and a distal end of the fluid supply passage 30 is provided with a nozzle 16 for discharging the fluid into the cavity 22 of the access assembly 1. Through this fluid supply passage 30, it is allowed to receive the fluid (for example, gas, liquid or gas-liquid two-phase mixture) from the outside of the access assembly 1, and to discharge the fluid into the cavity 22 of the access assembly 1 to clean the distal end part 18 of the surgical instrument 10. The fluid supply passage 30 is divided into a plurality of supply passage sections along the flow path. In this embodiment, preferably four supply passage sections are in fluid communication with each other, in which a first supply passage section 31 is arranged at the proximal end of the tube member, a second supply passage section 32 located downstream of the first supply passage section 31 is arranged in the conical portion of the tube member 3, a third supply passage section 33 located downstream of the second supply passage section 32 is arranged in the cylindrical portion of the tube member 3, and a fourth supply passage section 34 (i.e., the distal end member fluid channel 34) located downstream of the third supply passage section 33 is arranged in the distal end member 2. In this solution, the fourth supply passage section 34 has an inlet for inflow of fluid and a nozzle 16 for ejecting cleaning fluid into the cavity when cleaning the surgical instrument 10; and the fourth supply passage section 34 extends in a plane bounded by the central axis Z-Z' of the distal end member 2 and a ridge 13 correlated to the sealing valve 9. Fig. 2 shows a lateral top perspective view of a non-limiting embodiment of the distal end member 2 according to the present disclosure. As shown in the figure, the distal end member 2 includes a tubular housing 7 at its proximal side and four sealing valves 9 at its distal side. The tubular housing 7 is structured to be connected with the distal end of the access assembly 1 (as shown in Fig. 1), and a distal end of the tubular housing 7 is adjoined with the sealing valve 9. Two elastic pieces, namely a first elastic piece 23 and a second elastic piece 24 are arranged proximally from a proximal end of the tubular housing 7 along a direction of the central axis Z-Z' of the distal end member 2. The first elastic piece 23, together with the inner wall 6 of the tube member 3 of the access assembly, forms the third supply passage section 33. The first elastic piece 23 is structured to form, together with the tube member, the third supply passage section 33 of the fluid supply passage 30; and the second elastic piece 24 is structured to form, together with the tube member, a second fluid channel, especially a second fluid channel for carbon dioxide to flow into the abdominal cavity.

Each of the four sealing valves 9 is generally mountain-shaped, i.e., it's raised proximally in the middle along the circumferential direction thereof to form a "mountain" shape of the sealing valve 9, which is higher at the middle and lower at both sides, and also is higher at outer side and lower at inner side. The sealing valves 9 each include a ridge 13 extending along a radial direction of the tubular housing, as well as a first surface 11 and a second surface 12 on both radial sides of the ridge, respectively; and the nozzle is arranged on the ridge of one of these sealing valves 9. The first surface 11 and the second surface 12 are designed as planar surfaces in this embodiment, but they can also be designed as curved surfaces or a combination of planar surface and curved surface.

As shown in Figs. 2 to 5, the sealing valves 9 each have two radial edges 35 and one circumferential edge 36. Along the radial edge 35 of the sealing valve9, it's also provided with a scraping device 19 for scraping the liquid or tissue on the distal end part 18 of the surgical instrument. The scraping device 19 includes a boss 20 and a scraper 21. The boss 20 protrudes proximally from the sealing valve9 and extends along the radial edge 35 of the sealing valve 9; and the scraper 21 protrudes proximally from the boss 20 and extends along the radial axis of the boss 20.

A nozzle 16 is arranged on the sealing valve 9 away from the central axis Z-Z' of the distal end member 2. A radial distance between a center point of the nozzle 16 and the central axis Z-Z' of the distal end member 2 is greater than one quarter of an outer diameter of the tubular housing 7. Especially in the extreme state (where water droplets are not easy to remain on the distal end part of the surgical instrument), the radial distance between the center point of the nozzle 16 and the central axis Z-Z' of the distal end member 2 can also be equal to one quarter of the outer diameter of the tubular housing 7.

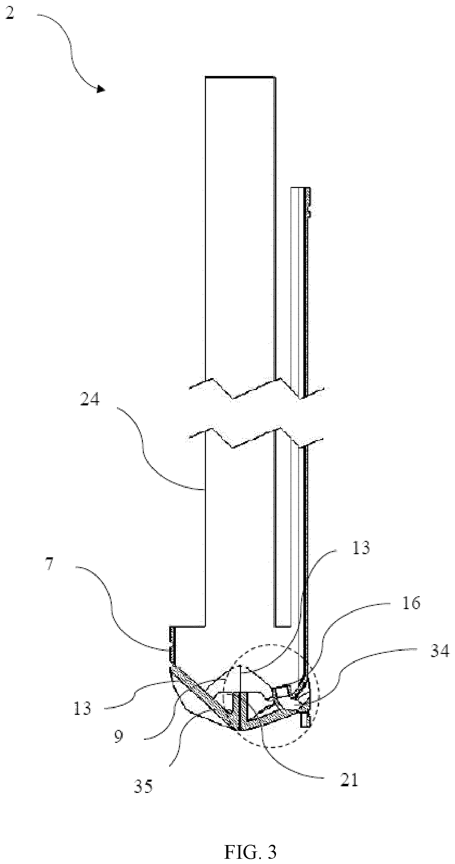

Fig. 3 shows a cross-sectional view of the distal end member 2 along line A-A (coincident with the ridge 13) in Fig. 2 according to an embodiment of the present disclosure. It can be further clearly seen from this figure that the distal end member fluid channel 34 is arranged on the ridge 13 of the sealing valve 9 away from the central axis Z-Z' of the distal end member 2, and extends in a plane bounded by the central axis Z-Z' of the distal end member 2 and the ridge 13.

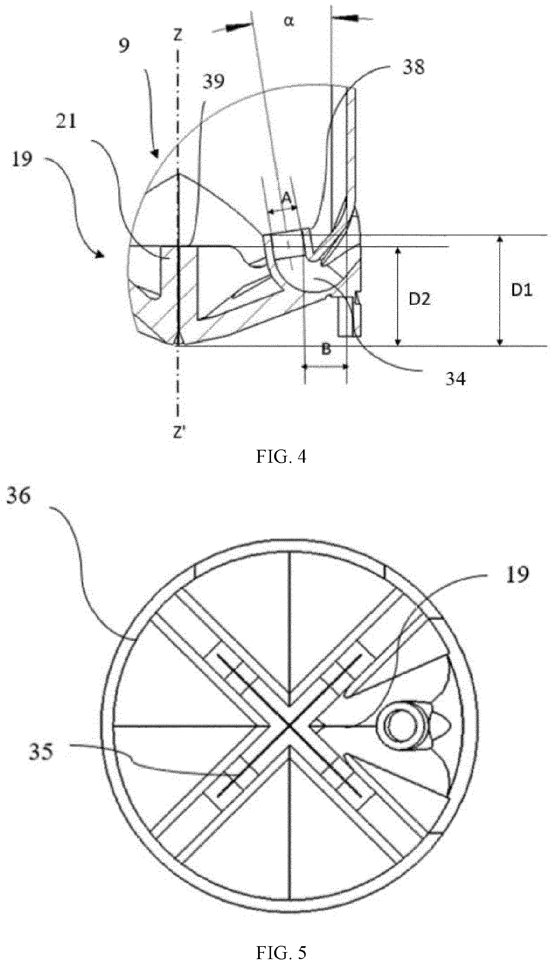

Fig. 4 shows a detailed view of the distal end member 2 indicated by the dotted circle in Fig. 3, in which a distal end of the third supply passage section 33 is communicated with the fourth supply passage section 34 (i.e., the fluid channel 34 of the distal end member 2), and a length of the third supply passage section 33 is about 100 mm. The nozzle 16 of the distal end member fluid channel 34 is configured to eject fluid towards a proximal direction of the access assembly 1 directionally, and the ejection direction of the nozzle is at an angle α with respect to a longitudinal axis of the distal end member 2, and a value of the angle α is about less than or equal to 60 degrees, preferably, the value of the angle α is about less than or equal to 15 degrees (the value of α is not 0 degree, and the value of α includes 15 degrees). In a case that the value of the angle α is greater than 60 degrees, the ejected fluid cannot be ejected onto the lens of the endoscope if the lens is facing away from the nozzle 16. In a case that the value of the angle α is less than or equal to 15 degrees, the ejected fluid can cover the access assembly at any axial rotation position of the tube member and realize the cleaning of the distal end part 18 of the surgical instrument 10 at any position. In a case that the value of the angle α is equal to 15 degrees, the fluid ejected from the nozzle 16 is exactly directed to the inlet 28 of the fluid discharge passage 37.

As shown in Fig. 4, a distance D1 between a proximal edge of the nozzle and a base of the distal end member is greater than a distance D2 between a proximal edge of the scraping device and the base of the distal end member. The base of the distal end member is a plane perpendicular to the longitudinal axis of the distal end member, and the most distal point of the distal end member 2 lies in this plane. The proximal edge 38 of the nozzle 16 is higher than the proximal edge of the scraper 21 in the drawings' plane, in other words, the proximal edge 38 of the nozzle 16 is located more proximally than the proximal edge 39 of the scraper 21, so that the nozzle 16 is in contact with the distal end part of the surgical instrument earlier than the scraper contacts the distal end part, under a negative pressure. During the distal end part 18 of the surgical instrument 10 retracting from the distal end member 2 proximally, the sealing valve 9 is at its beginning of an open state; at this time, due to the action of a negative pressure, the gas in the abdominal cavity flows into the tube member 3 of the access assembly through a gap, and the water on the distal end part 18 of the surgical instrument 10 will be taken away with the air flow. If there is a lot of water on the distal end part 18 of the surgical instrument 10, the suction effect of the negative pressure will not be enough to remove all the water, resulting in water droplets remaining on the distal end part 18 of the surgical instrument 10. Since the proximal edge 38 of the nozzle 16 is higher than the proximal edge 39 of the scraper 21 in Fig. 4, the distal end part 18 will block the nozzle 16 before it's in contact with the scraper 21 during the surgical instrument 10 moving distally, thus reducing the amount of water ejected onto the distal end part 18 of the surgical instrument 10. As a result, less water needs to be taken away by the suction effect of the negative pressure, so that water droplets are not easy to remain on the distal end part 18 of the surgical instrument 10, and the effect of removing the water droplets is better.

A distance B between an inner edge of the nozzle 16 away from the central axis Z-Z' of the distal end member 2 and an outer wall of the tubular housing 7 or an inner wall 6 of the tube member 3 of the access assembly is greater than an inner diameter A of the nozzle 16, whereby the wall-adhering phenomenon can be well avoided. The wall-adhering phenomenon refers to that the jet ejected from the nozzle 16 is not in an ejected state but flows along the inner wall 6 of the tube member 3 of the access assembly. The occurrence of this phenomenon will result in that the jet flow cannot be effectively ejected onto the distal end part 18 of the surgical instrument 10, thus adversely affecting the cleaning effect.

Fig. 5 shows a top view of the distal end member 2 in Fig. 2, from which it can be clearly seen that the four sealing valves 9 are distributed symmetrically about the central axis, and that the surfaces 11, 12 (not labeled) located at both radial sides of the ridge 13 are also symmetrically arranged. The nozzle 16 of the distal end member fluid channel 34 is located exactly on the ridge 13, and the radial distance between the center point of the nozzle 16 and the central axis Z-Z' of the distal end member 2 is significantly greater than one quarter of the outer diameter of the tubular housing 7.

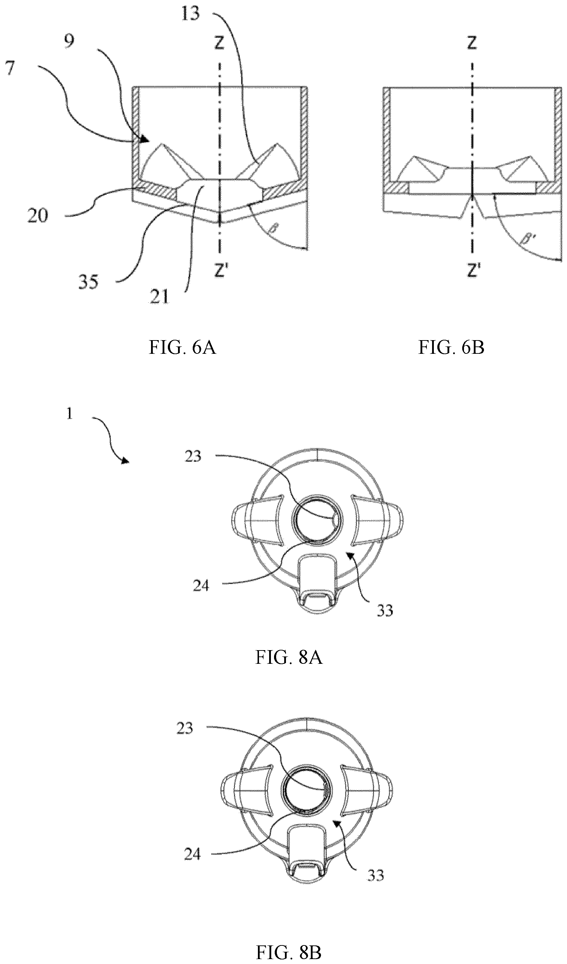

Figs. 6A-6B show cross-sectional views of two different embodiments of the distal end member 2 taken along line B-B in Fig. 2, where line B-B corresponds to the radial edge 35 of the sealing valve9. The difference between these two embodiments lies in the different angles β, β' between the radial edge 35 and the central axis Z-Z' ' of the distal end member 2. The angle β of an embodiment of the present disclosure shown in Fig. 6A is smaller than the angle 0' of another embodiment shown in Fig. 6B. In order to obtain a better cleaning effect, a larger washing force is usually required, so the sealing valve9 should be able to bear a larger negative pressure (provided by the vacuum suction source of the access assembly). If the smaller angle β is selected, the distal end member 2 shown in Fig. 6A according to the present disclosure will bear a larger negative pressure than the distal end member 2 in another embodiment of Fig. 6B. However, in order to ensure that the distal end part 18 of the surgical instrument 10 can be touched under a negative pressure when the surgical instrument 10 passes through the sealing valve 9, the corresponding scraping device 19 in Fig. 6A also needs to be designed higher than that in the embodiment of Fig. 6B accordingly.

Figs. 7A-7B are lateral cross-sectional views of the access assembly 1 with a distal end member 2 according to different embodiments during cleaning. As an example, when the distal end portion 18 of the surgical instrument 10 is covered by smoke, condensate, tissue, blood and other body fluids in a patient's body cavity and hence the surgical field is affected during a surgery, the access assembly 1 according to the present disclosure can be used to perform an intraoperative cleaning process without the need of taking the entire surgical instrument 10 out of the access assembly 1. During the cleaning process, first of all, the distal end part 18 of the surgical instrument 10 is retracted proximally into the cavity 22 of the access assembly 1 composed of the tube member 3, the proximal sealer 25 and the distal end member 2 which are sealed with each other. As shown in Fig. 7A, the fluid 15 is ejected from the nozzle 16 of the distal end member 2, where the value of the angle α is less than 15 degrees. The ejected fluid can effectively cover the whole tube member section of the access assembly 1 from the inlet 28 of the fluid discharge passage 37 to the distal end member 2, with a length of about 100 mm, so as to realize cleaning at any position and any angle within the cavity 22 of the tube member section. In a case that the nozzle 16 is oriented at an angle α', as shown in Fig. 7B, in this embodiment, if the value of the angle α' is 15 degrees, the fluid 15 ejected from the nozzle 16 is directed to the inlet 28 of the fluid discharge passage; and if the value of the angle α is 0-15 degrees (the value of α is not 0 degree, and the value of α includes 15 degrees), the ejected fluid can cover and clean any position in the cavity 22 of the tube member of the access assembly. If the angle α is greater than 15 degrees and less than 60 degrees, the position where the ejected fluid is ejected into the tube member is lower than the inlet 28 of the fluid discharge passage; in this case, although it cannot realize cleaning the distal end part 18 of the surgical instrument 10 at any position and any angle in the cavity 22 of the tube member section, the cleaning can still be carried out as long as the distal end part 18 of the surgical instrument 10 moves distally appropriately.

Figs. 8A-8B are top views of the access assembly 1 with the first elastic piece 23 in a first state and a second state, respectively. The third supply passage section 33 is surrounded by the cylindrical portion of the tube member, the elastic piece 23 and optionally a sealing body, in which the first elastic piece 23 can be configured to bend radially outwards relative to the central axis Z-Z' of the tube member 3 in the first state, and to bend radially inwards and at least partially approach the central axis Z-Z' of the tube member 3 in the second state.

The third supply passage section 33 can be changed from a first state to a second state due to a pressure difference between the pressure in the cavity 22 and the pressure in the third supply passage section 33, wherein the elastic piece 23 is in the first state when the pressure in the cavity 22 of the access assembly 1 is not lower than that in the third supply passage section 33, and the elastic piece 23 is in the second state when the pressure in the cavity 22 of the access assembly 1 is lower than that in the third supply passage section 33.

When the surgical instrument 10 is to be cleaned, a vacuum suction device is connected to the vacuum suction interface 27 of the access assembly 1, and a negative pressure causes a suction effect to the cavity 22 of the access assembly 1; then a fluid source is connected to the fluid inlet 5, and pressurized fluid flows into the third supply passage section 33, so that the pressure in the third supply passage section 33 is greater than that in the cavity 22 of the access assembly 1. Due to this pressure difference, the elastic piece 23 constituting the third supply passage section 33 is deformed from the first state shown in Fig. 8A to the second state shown in Fig. 8B, that is, it is deformed from bending radially outwards with respect to the longitudinal axis Z-Z' of the tube member 3 to bending radially inwards with respect to the longitudinal axis Z-Z' of the tube member 3 as shown in Fig. 8B, thereby increasing a cross-sectional area of the third supply passage section 33 in the section perpendicular to the longitudinal axis Z-Z' of the access assembly 1 (as shown in Fig. 8B). The increase in the cross-sectional area of the third supply passage section 33 in turn leads to a decrease in the resistance of fluid flowing through the third supply passage section 33. Under the condition that the pressure of the fluid source is constant, this will increase the flow rate of the fluid, thereby enhancing the cleaning force of the fluid on the distal end part of the surgical instrument 10 or the speed of discharging gas such as CO₂ into the body cavity of the patient.

Alternatively, under the condition that the flow rate of fluid is constant, the requirement for pressurizing the fluid source can be lowered due to the reduced resistance. In this case, common equipment in an operating room, such as physiological saline bottle, can be used as the fluid source without the need of providing an additional pressure source, such as a pump. The elastic piece 23 and/or the elastic piece 24 may be made of a medical-grade material with an elastic modulus lower than that of the tube member 3, for example, a medical silica gel material, so as to be deformed at a lower pressure difference without significantly affecting the shape of the tube member 3.

## Claims

1. An access assembly for a surgical instrument, comprising:
a tube member structured to extend along a longitudinal axis of the access assembly and configured to receive the surgical instrument;
a proximal sealing device configured to connect the surgical instrument to a proximal end of the tube member in a sealed manner;
a distal end member connected with the tube member, the distal end member comprising: a tubular housing structured to be connected with a distal end of the tube member; and a plurality of sealing valves arranged at a distal end of the tubular housing and configured to be openable and closable to open and seal a distal end of the access assembly, at least one of the plurality of sealing valves comprising a nozzle for ejecting a fluid, a fluid inlet for receiving the fluid, and a distal end member fluid channel for fluidly communicating the fluid inlet and the nozzle; wherein the nozzle is configured to eject the fluid towards a proximal direction of the access assembly directionally, and an ejection direction of the nozzle has an angle α with respect to a longitudinal axis of the distal end member;
a fluid supply passage configured to provide the fluid to the access assembly; and
a fluid discharge passage configured for the access assembly to discharge the fluid, wherein the fluid discharge passage comprises an outlet and an inlet, and the inlet of the fluid discharge passage is arranged at the proximal end of the tube member.

2. The access assembly according to claim 1, wherein a value of the angle α is less than or equal to 60 degrees, preferably, the value of the angle α is less than or equal to 15 degrees.

3. The access assembly according to claim 2, wherein the value of the angle α is equal to 15 degrees; and/or, the nozzle is directed to the inlet of the fluid discharge passage.

4. The access assembly according to any one of claims 1-3, wherein a scraping device for scraping a distal end part of the surgical instrument is arranged on the sealing valve, and the nozzle is arranged on at least one of the plurality of sealing valves away from the scraping device;
the scraping device is at least partially arranged at a central axis of the distal end member.

5. The access assembly according to any one of claims 1 to 3, wherein a radial distance between a center point of the nozzle and a central axis of the distal end member is greater than or equal to one quarter of an outer diameter of the tubular housing.

6. The access assembly according to any one of claims 1-3, wherein each of the plurality of sealing valves comprises a ridge extending radially along the tubular housing and surfaces on both radial sides of the ridge,
the nozzle is arranged on one of a plurality of the ridges, and the distal end member fluid channel extends in a plane bounded by the longitudinal axis of the distal end member and the ridge.

7. The access assembly according to any one of claims 1 to 3, wherein a distance between an inner edge of the nozzle away from a central axis of the distal end member and an outer wall of the tubular housing is greater than an inner diameter of the nozzle.

8. The access assembly according to claim 4, wherein a distance between a proximal edge of the nozzle and a base of the distal end member is greater than a distance between a proximal edge of the scraping device and the base of the distal end member.

9. The access assembly according to claim 4, wherein the scraping device protrudes proximally so that it can be in contact with the distal end part of the surgical instrument in a case where the surgical instrument passes through the sealing valve.

10. The access assembly according to claim 4, wherein the plurality of sealing valves are arranged along a circumferential direction of the tubular housing, each of the plurality of sealing valves comprises two radial edges and one circumferential edge, and the scraping device proximally protrudes from a surface of the sealing valve.

11. The access assembly according to claim 4, wherein the scraping device comprises at least one boss, which protrudes proximally from the sealing valve and is arranged on the sealing valve along at least one radial edge of the sealing valve, and
the scraping device further comprises at least one scraper, which protrudes proximally from a portion of the boss close to the longitudinal axis of the distal end member.

12. The access assembly according to claim 1, wherein the distal end member at least is connected with one elastic piece, and the elastic piece is structured to form, together with a part of the tube member, at least part of the fluid supply passage, and
at least a distal end of the fluid supply passage is communicated with the distal end member fluid channel.

13. The access assembly according to claim 1, wherein the surgical instrument is an endoscope provided with a lens at a distal end part thereof.
